# EUROPEAN PATENT SPECIFICATION

(11) **EP 1 949 863 B1**
(45) Date of publication and mention of the grant of the patent: **19.10.2011**
(21) Application number: 06126186.3
(22) Date of filing: 14.12.2006
(51) Int. Cl.: A61B 17/28, A61B 17/04, A61B 17/00, A61B 17/22

(54) **A tissue clamp for endolumenal local excision of tissue**
Gewebeklammer zur endoluminalen, lokalen Gewebeexzision
Pince pour excision locale endoluminale de tissu

(43) Date of publication of application: 30.07.2008
(73) Proprietor: ETHICON ENDO-SURGERY, INC., Cincinnati, OH 45242-2839 (US)
(72) Inventor: Thompson, Brian James, Cincinnati, OH 45226 (US)
(74) Representative: Leihkauf, Steffen Falk

(56) References cited:
- WO-A-2004/110285
- WO-A-2006/122279
- JP-A- 2004 000 601
- US-A1- 2005 119 524
- US-A1- 2006 241 692
- US-B1- 7 118 528

## Description

The invention relates in general to surgical instruments for the localized treatment of polyps, cancers and suspicious lesion in natural ducts of the body of a patient. Particularly, the invention relates to a surgical device to enable invagination and holding of a tissue portion for a full thickness local excision of a polyp or other pathologically altered tissue by endolumenal surgery.

The most commonly performed surgical treatment of cancers involving natural ducts, such as colon cancer or small bowel cancer, is called segmental resection and involves general anesthesia, an incision in the abdomen, the removal of the cancer and a length of normal colon or bowel on either side of the cancer, as well as the nearby lymph nodes. The segmental resection is rather invasive and the post-operative course of the patient is comparatively long.

Very early colon or small bowel cancers, classified as stage 0 and stage I tumors, or cancerous polyps which have not yet involved the nearby lymph nodes can also be removed by endolumenal surgery. This surgical method, called polypectomy, is performed through a colonoscope or gastroscope without the need to cut into the abdomen. The operational site is accessed endolumenally and, in case of cancerous polyps, the cancer is cut out across the base of the polyps stalk, while in case of superficial cancers, the cancer itself and a small amount of nearby tissue is removed by local excision, e.g. by means of an endolumenal stapling device

Such an endolumenal stapling device comprises a staple fastening assembly with a proximal cartridge device and a distal anvil movable relative to the cartridge device. The cartridge device houses a staple- and knife-driving mechanism adapted to push two or more rows of staples and a knife out of a distal end surface of the cartridge device and towards the anvil. The anvil comprises a staple forming surface and a cutting block matching the staple rows and the knife of the cartridge device and is adapted to cooperate with the cartridge device for forming the ends of the staples exiting from the cartridge device and for excising the lesion tissue by the knife. In order to position the lesion, e.g. polyp or superficial cancer and the surrounding tissue between the cartridge device and the anvil of the stapler, it is necessary to place a suture or to apply a surgical grasper to the lesion and to pull the tissue by the suture or grasper inside the staple fastening assembly of the stapler. Although the use of endolumenal surgical stapling instruments is beneficial and greatly facilitates the performance of a polypectomy, it still involves some problems.

Due to the encumbrance of the cartridge device of the stapler, it is often very difficult to place a suture or to apply a grasper to the tissue to be excised and to endoscopically visualize and control the positioning of the tissue within the instrument. This leads to an increased risk of colon or bowel wall damages, e.g. when too much tissue is drawn into the device and excised, and to a risk of incomplete removal of the lesion when only part of the pathologically altered tissue has been placed beyond the staple line. Moreover, the bulky structure of the endolumenal staplers render it difficult to transport further surgical instruments to the operational site.

Last but not least, surgical staplers are rather complex instruments which combine and perform several functions or procedural steps of a polypectomy, i.e. tissue positioning (clamping), tissue fixation (stapling) and tissue excision (cutting). While such a single device approach is certainly generally desirable, it entails comparatively high instrument costs and the impossibility of varying single steps of the endolumenal treatment of polyps or cancers and of using the surgical techniques which are considered the most appropriate for the patient (e.g. tissue fixation by suturing instead of stapling or tissue excision by radiofrequency, needle knife or ultrasonic energy instead of cutting). US-A-2005/0119524 and WO 2004/110285 disclose examples of prior art devices having the features of the preamble of claim 1.

In view of the drawbacks of the prior art, the object of the invention is to provide a surgical instrument for performing an endolumenal excision of a lesion, particularly of a cancer or polyp, which permits a good endoscopic visual control of the position and amount of tissue to be fixated and excised, in order to minimize the risk of incomplete removal of the lesion and of lumen wall damages.

Within the general scope of the main object, it is a further aim of the present invention to provide a surgical device to enable invagination of tissue within a flexible endoscopic clamp for a full thickness local excision of a polyp, suspicious lesion etc.

It is a further aim of the present invention to provide a simply structured low-cost alternative to complex surgical staplers which can be used in conjunction with other available surgical tools and techniques in order to enable the surgeon to freely and selectively chose the most appropriate approach to excise the lesion. These and other objects are achieved by a surgical tissue clamp for endolumenally holding and invaginating a portion of tissue in a polypectomy or generally in an excision of a lesion according to claim 1. The dependent claims cover advantageous embodiments of the invention.

According to the invention, the surgical tissue clamp comprises a flexible elongate insertion shaft, a handle arranged at a proximal end of the insertion shaft and a clamping head arranged at a distal end of the insertion shaft. The clamping head comprises a proximal jaw and a distal jaw movable relative to the proximal jaw and configured to cooperate with the proximal jaw for clamping the tissue in a substantially radial orientation with respect to a longitudinal axis of the clamping head. An actuating device is operatively connected with said proximal and distal jaws and adapted to approximate the jaws and to hold them in a tissue clamping configuration. The proximal and distal jaws have a curved configuration such that they extend at least approximately along the circumference of an imaginary circle or ellipse which is preferably perpendicular to the longitudinal axis of the clamping head.

Thanks to the curved shape of the jaws, the clamping head adapts well to the shape of the natural duct, doesn't obstruct the visual field of an endoscope and can be moved along and relative to the endoscope through which additional instruments can be delivered to the operational site.

Preferably, the proximal jaw comprises a curved clamping (ring-) segment (in the following referred to as proximal clamping segment) having a very small radial thickness so that it determines a thin clamping line and obviates undesired obstruction of the natural duct at the operational site. Thanks to the small radial thickness of the proximal clamping segment, the tissue immediately adjacent to the clamping line can be comfortably reached by the above mentioned additional instruments from a proximal radial outside and from a proximal radial inside of the jaws. The small radial thickness of the clamping segments further allows additional instruments to be moved from a proximal side astride the clamping segments to a distal side thereof in order to manipulate, e.g. grasp or push or suture the clamped or invaginated tissue also from the distal side. With the known endoluminal staplers, such a distal approach was hampered by the radial bulkiness of the staple cartridge. Advantageously the mean radial thickness of the proximal clamping segment is less than 1/8 of its bending radius, preferably less than 1/10 of its bending radius thereby allowing good visual and instrumental access to the tissue to be clamped or invaginated.

Apart from having a small radial thickness, according to a preferred embodiment, the proximal clamping segment has also a small longitudinal (distal-proximal) thickness, thereby enabling the surgeon to comfortably reach and manipulate both the tissue radially outside and immediately adjacent to the clamping segment and the tissue volume held radially inside the clamping segment. Preferably, the longitudinal thickness of the proximal clamping segment is less than 1/2 of its bending radius, preferably less than 1/5 of its bending radius.

In accordance with a further aspect of the invention, the clamping head defines at least one proximal access aperture configured to access the space, i.e. the tissue, radially inside the jaws and that radially outside the jaws directly across the clamping head, thereby obviating the need to withdraw an instrument proximally from the clamping head and externally bypass the latter in order to reach the tissue situated radially outside the jaws. This makes it possible to obtain a precise positioning of the tissue to be clamped and invaginated as well as a fast access to different places of the operational site by additional instruments once the tissue clamp has been applied, e.g. by tissue fixation and excision devices.

In accordance with an advantageous embodiment, the slender proximal clamping segment is connected to the insertion shaft by a proximal spacing frame which delimits together with the clamping segment the above said proximal access aperture.

According to one aspect of the invention, the proximal spacing frame comprises a substantially U-shaped frame having two longitudinal and approximately parallel opposite legs which are distally connected to the lateral ends of the proximal clamping segment as well as a curved proximal base spoke which connects the U-shaped frame to the insertion shaft. In this way, the proximal access aperture defined by the proximal clamping segment and the longitudinal legs of the proximal spacing frame consent to endoscopically visualize and to manipulate the tissue proximally adjacent the clamped or invaginated tissue directly across the clamping head. Moreover, the proximal U-shaped frame together with the proximal clamping segment provide a cage-like spacing feature acting both radially and proximally, thereby providing space both for the tissue volume held radially inside the clamping line and for other surgical instruments which can be endolumenally inserted to manipulate the clamped or invaginated tissue while it is held in position by the clamping head.

According to a preferred embodiment, the proximal base spoke has substantially the same curvature as the proximal clamping segment, so that the U-shaped spacing frame and the proximal clamping segment form a longitudinally straight and transversally curved rectangular frame, such as a cylindrical halve shell frame or halfpipe frame, defining internally the above said rectangular proximal access aperture.

Like the proximal jaw, also the distal jaw comprises a curved clamping (ring-) segment (in the following referred to as distal clamping segment) complementary to the proximal clamping segment of the proximal jaw and connected by means of a distal spacing frame to a pulling rod received in the insertion shaft and operatively connected to the actuating device of the tissue clamp.

Also the distal clamping segment has a small radial thickness so that it determines a thin clamping line and obviates undesired obstruction of the natural duct at the operational site. Thanks to the small radial thickness of the distal clamping segment, the tissue immediately distally adjacent to the clamping line can be comfortably reached by the above mentioned additional instruments from a proximal side of the clamping head. Advantageously the mean radial thickness of the distal clamping segment is less than 1/8 of its bending radius, preferably less than 1/10 of its bending radius thereby allowing good instrumental access to the tissue to be clamped or invaginated.

Apart from having a small radial thickness, according to a preferred embodiment, the distal clamping segment (just like the proximal clamping segment) has also a small longitudinal (distal-proximal) thickness, thereby enabling the surgeon to comfortably reach and manipulate both the tissue radially outside and immediately adjacent to the distal clamping segment and the tissue volume held radially inside the clamping head. Preferably, the longitudinal thickness of the distal clamping segment is less than 1/2 of its bending radius, preferably less than 1/5 of its bending radius.

In accordance with an advantageous embodiment, the slender distal clamping segment is connected to the pulling rod by a distal spacing frame longitudinally (in a proximal-distal direction) and laterally overlapping the proximal spacing frame. The distal spacing frame and the distal clamping segment define a window opening which overlaps the above mentioned proximal access aperture of the proximal jaw.

It is further advantageous to give also the distal spacing frame a substantially U shaped or quadrangular shape with two longitudinal approximately parallel opposite legs distally connected to the lateral ends of the distal clamping segment and a curved proximal base spoke connecting the longitudinally straight and transversally curved U-shaped or quadrangular frame to the pulling rod, thereby adapting the clamping head to the duct wall and leaving a free passage space for visualization and instrument access to the clamped tissue. The distal spacing frame is advantageously configured such that its longitudinal legs are close to, preferably overlapping the longitudinal legs of the proximal spacing frame in order not to obstruct the proximal access aperture defined by the latter. Advantageously, the distal spacing frame is arranged radially inside the proximal spacing frame such that during the approximation of the jaws, the proximal spacing frame can be held in static contact against the lumen wall while the distal spacing frame slides along the proximal spacing frame without rubbing the lumen wall.

Even though not indispensable, in order to uniformly distribute the clamping force it is preferable to connect the insertion shaft and the pulling rod to the vertexes of the arch-shaped proximal and distal jaws half way between their lateral ends.

According to a further aspect of the invention, the actuating mechanism comprises a manual actuating member arranged at the handle so that it can be actuated extracorporeally, and a moving mechanism operatively connected to the manual actuating member and to the distal and proximal jaws and adapted to approximate (and preferably also to separate) the jaws in response to an actuating movement of the manual actuating member. Advantageously, a locking mechanism is also operatively connected to the distal and proximal jaws and configured to lock the jaws in their tissue clamping configuration. thereby setting both hands of the surgeon free and render them available to further manipulate the clamped tissue with other instruments.

In order to allow the locking mechanism to be accommodated inside a portion of the clamping head or insertion shaft without substantially increasing the dimension of the latter, it proved advantageous to embody the actuating mechanism with a jackscrew which is rotatable but translationally locked with respect to the proximal jaw, a threaded connecting portion of the pulling rod of the distal jaw meshing the jackscrew, a rotating knob mounted on the handle and torsionally coupled with the jackscrew by a flexible rotary rod such that a rotation of the rotating knob causes the jackscrew to screw on the pulling rod thereby approximating or, viceversa, separating the jaws. In this way, the frictional resistance in the screw connection between the jackscrew and the pulling rod provides the above mentioned jaw locking feature. This frictional locking capability is further increased by the longitudinal clamping force which causes a mutual pre-stress of the threads that can only be overcome by actively forcedly rotating the jack-screw.

The rotary rod can comprise e.g. a torsionally stiff single flexible structure all along (torsion cable) or a structure comprising flexibly linked short rigid members or a tightly wound helix.

The device of the invention can be use in a method for endolumenal full thickness local excision of a polyp or lesion comprising the general steps of endolumenally accessing the operational target site, clamping and holding the tissue to be excised by the surgical tissue clamp according to the invention, fixating and securing the tissue in the sense of a non-circumferential anastomosis by an instrument different from the surgical tissue clamp and excision of the lesion or polyp by an instrument different from the surgical tissue clamp in order to enable the surgeon or gastroenterologist to chose for each single step of the method the most appropriate surgical technique according to his skills and preferences and according to the specific anatomical and pathological situation of the patient.

These and other details and advantages of the present invention shall be made apparent from the accompanying drawings and the description thereof, which illustrate embodiments of the invention and, together with the general description of the invention given above, and the detailed description of a preferred embodiment of the invention given below, serve to explain the principles of the present invention.
- Figure 1 is a lateral isometric view of a surgical tissue clamp according to an embodiment of the invention;
- Figure 2 is an enlarged longitudinally sectioned view of a detail of an actuating device of the surgical tissue clamp of figure 1;
- Figure 3 is an isometric view of the clamping head of the surgical tissue clamp during clamping and invaginating a tissue portion;
- Figure 4 is an isometric view of the clamping head of the surgical tissue clamp of figure 1 invaginating the tissue portion and of a tissue apposition device piercing through the invaginated tissue before applying a T-Tag fastener;

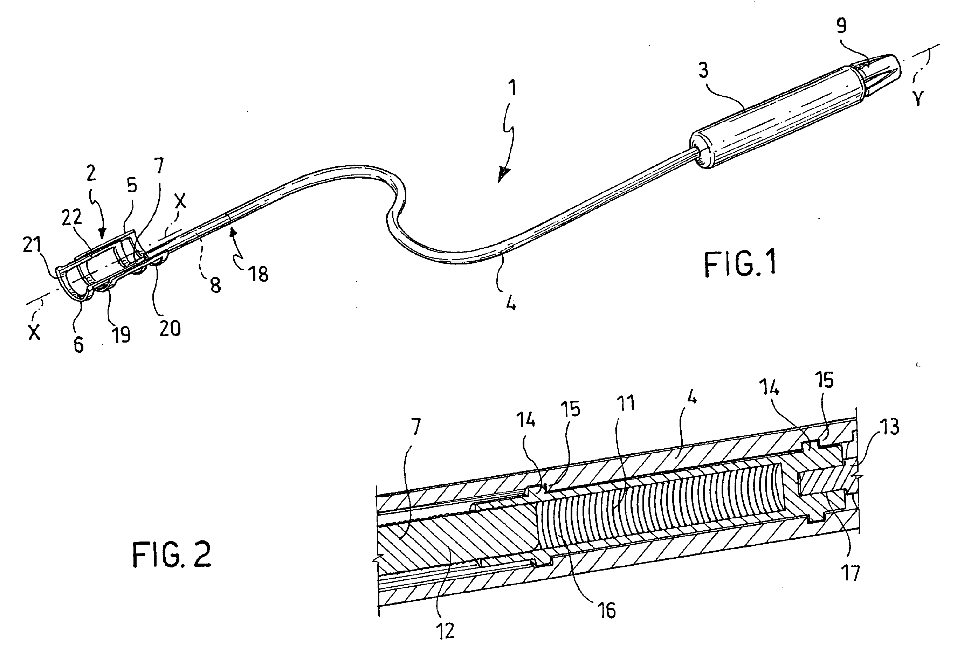

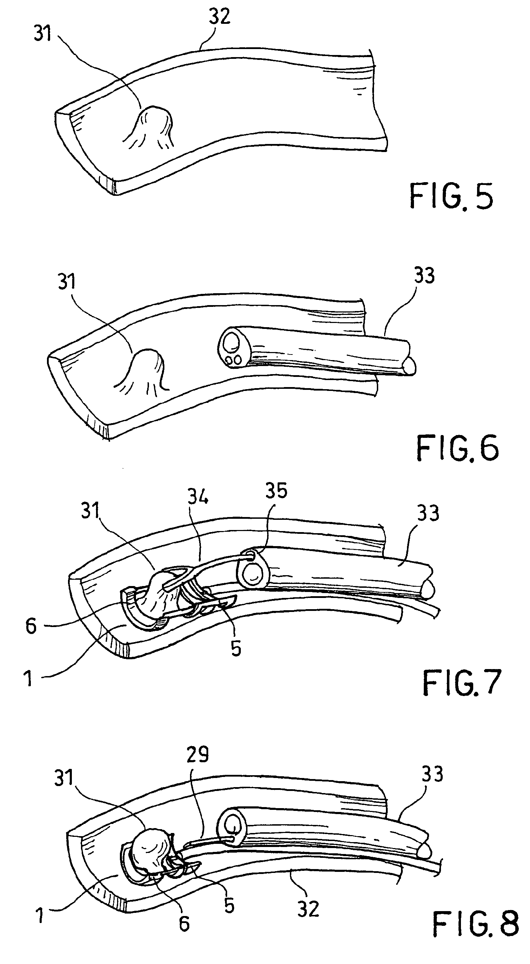

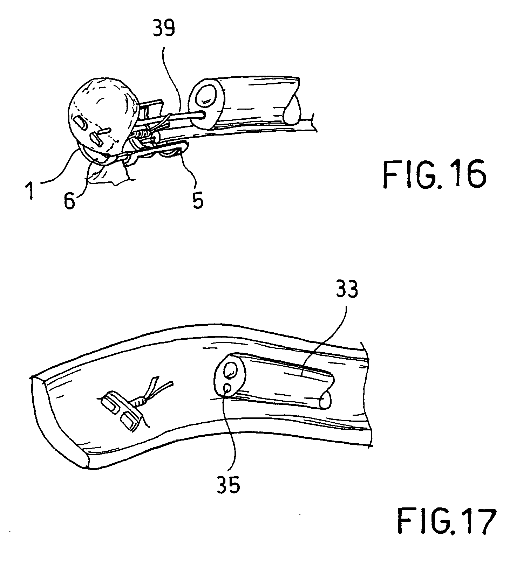

- Figures 5 to 17 are a schematic illustration of a surgical method for an endoluminal local excision of a polyp using the surgical tissue clamp of figures 1 to 4. Turning to the figures, fig. 1 is an isometric overall view of a surgical tissue clamp 1 according to a preferred embodiment. The tissue clamp 1 comprises, in its distal end region, a clamping head 2 and, in its proximal end region, a handle 3. The handle 3 and the clamping head 2 are connected via an elongate flexible insertion shaft 4 adapted to endolumenally insert and position the clamping head 2 by manually operating the handle 3 which is intended to remain outside the body of the patient.

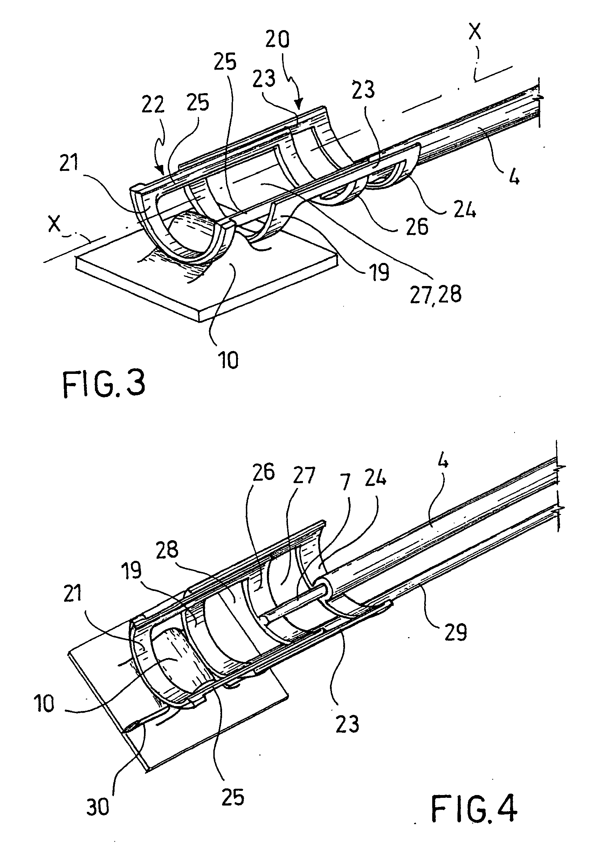

The clamping head 2 include a proximal jaw 5 rigidly connected to the distal end of the tubular insertion shaft 4 and a distal jaw 6 connected to a distal end of a pulling rod 7 whose proximal end is slidingly housed inside the distal end of the insertion shaft 4 such that the jaws can be translated relative to another and cooperate for clamping the tissue around a polyp or lesion intended to be excised in a substantially radial orientation with respect to a longitudinal axis X of the clamping head 2. The tissue clamp 1 further comprises an actuating device 8 operatively connected with the proximal and distal jaws 5, 6 and with a rotating knob 9 arranged at a proximal end of the handle 3 such that manual actuation of the rotating knob 9 causes the jaws 5, 6 to approximate and, hence, to clamp the tissue 10 (Figures 3, 4). The actuating device 8 is also adapted to lock the jaws in their mutual position when the rotating knob 9 is not further moved or held in order to automatically hold the jaws in their tissue clamping or invaginating configuration. According to an advantageous embodiment, the actuating device is configured to allow the jaws to depart from another (e.g. in response to a reverse movement of the rotating knob) in order to release the clamped tissue 10, even though this feature might not be indispensable in cases in which the excised tissue volume can be removed directly together with the clamp 1 when it is still clamped.

According to a preferred embodiment (Figure 2) the actuating device 8 comprises a jackscrew 11 received in the distal end of the insertion shaft 4 adjacent to the clamping head 2. The jackscrew 11 is rotatable but translationally locked with respect to the proximal jaw 5 and meshes with a threaded connecting portion 12 of the pulling rod 7. The rotating knob 9 mounted on the handle 3 is torsionally coupled to the jackscrew 11 by a flexible rotary rod 13, e.g. a torsion cable, such that a rotation of the rotating knob 9 causes the jackscrew 11 to screw on the pulling rod 7 (which is rotationally constrained so that it cannot rotate together with the jackscrew) thereby approximating or separating the jaws 5, 6.

The jackscrew 11 comprises an external shape of a body of revolution (with respect to the longitudinal axis of the insertion shaft 4) with two annular flanges 14 engaging corresponding internal annular recesses 15 of the distal end portion of the insertion shaft 4 in order to provide the above said rotatable but axially locked support. The jackscrew 11 defines a distally open and internally threaded hole 16 adapted to receive the externally threaded proximal connecting portion 12 of the pulling rod 7 and a preferably polygonal proximal seat 17 for detachably engaging the distal end of the rotary rod 13 having complementary shape, such that the jackscrew 11 can be torsionally coupled and detached from rotary rod 13. The detachable coupling between the rotary rod 13 and the jackscrew 11 together with a detachable connection 18 between the insertion shaft 4 and its distal end portion which remains attached to the clamping head and houses the jackscrew 11 enable the single parts of the surgical clamp to be separately and selectively disinfected and disposed and reused.

Rotary rod 13, e.g. a torsionally stiff single torsion cable or a structure comprising flexibly linked short rigid members or a tightly wound helix, extends from jackscrew 11 proximally throughout the insertion shaft 4 and the handle 3 and its proximal end is coupled to rotate with the rotating knob 9 which is arranged at the proximal end of handle 3, preferably coaxially to a longitudinal handle axis Y.

In order to better access and visualize the operational site, the proximal and distal jaws 5, 6 have a curved configuration such that they extend at least approximately along the circumference of an imaginary circular or elliptic cylinder constructed around the longitudinal axis X of the clamping head 2. Particularly, the proximal jaw 5 comprises a curved proximal clamping (ring-) segment 19 which determines a curved tissue clamping line and which is rigidly connected to the insertion shaft 4 by a proximal spacing frame 20. Similarly, also the distal jaw 6 comprises a curved distal clamping (ring-) segment 21 complementary to the proximal clamping segment 19 of the proximal jaw 5 and connected by means of a distal spacing frame 22 to the pulling rod 7 received by the insertion shaft 4.

Advantageously, both the curved proximal clamping segment 19 and the curved distal clamping segment 21 have very small mean radial thicknesses of less than 1/8 of their bending radius, preferably less than 1/10 of their bending radius and, preferably, also very small longitudinal (proximal - distal) thicknesses of less than 1/2 of their bending radius, preferably less than 1/5 of their bending radius.

The proximal spacing frame 20 comprises a substantially U-shaped frame having two longitudinal and approximately parallel opposite legs 23 distally connected to the lateral ends of the proximal clamping segment 19 and a curved proximal base spoke 24 connecting the U-shaped frame to the insertion shaft 4. The distal spacing frame 22 overlaps the proximal spacing frame 20 and comprises also a substantially U shaped or quadrangular frame with two longitudinal approximately parallel opposite legs 25 distally connected to the lateral ends of the curved distal clamping segment 21 of the distal jaw and a curved proximal base spoke 26 connecting the U-shaped or quadrangular distal frame to the pulling rod 7. The longitudinal legs 25 of the distal spacing frame 22 are arranged very close to, preferably overlap the longitudinal legs 23 of the proximal spacing frame 20 in order not to obstruct a proximal access aperture 27 defined by the proximal spacing frame 20 and the proximal clamping segment 19 and configured to access the space radially inside the jaws 5, 6 and the space radially outside the jaws 5, 6 directly across the clamping head 2.

As can be seen in figures 3 and 4, a window opening 28 defined by the longitudinal legs and the curved clamping segment of the distal jaw 6 is overlapping the proximal access aperture 27 of the distal jaw 6 in order to assure both a direct visualization and a direct instrumental access across the clamping head 2.

The proximal and distal spacing frames 20, 22 are both longitudinally straight and transversally curved in a complementary manner such that they provide a cylindrical halve shell frame or halfpipe frame defining the proximal access aperture 27 and acting like a tissue supporting cage adapted to keep the operational site both laterally and longitudinally clear and unobstructed.

Figures 5 to 17 illustrate a method for an endolumenal full thickness local excision of a lesion or polyp which involves the use of the surgical tissue clamp 1.

In general terms, the method involves the following steps:
- Endolumenally accessing the operational target site, e.g. a lesion or polyp 31 in the colon wall 32 or in the small bowel wall.
- Clamping and holding the tissue to be excised by the surgical tissue clamp 1 according to the invention.
- Fixating and securing the tissue in the sense of a non-circumferential anastomosis using instruments different from said surgical tissue clamp.
- Excision of the lesion or polyp 31 using an instrument different from said surgical tissue clamp 1.

### Endolumenally accessing and clamping or invaginating the tissue

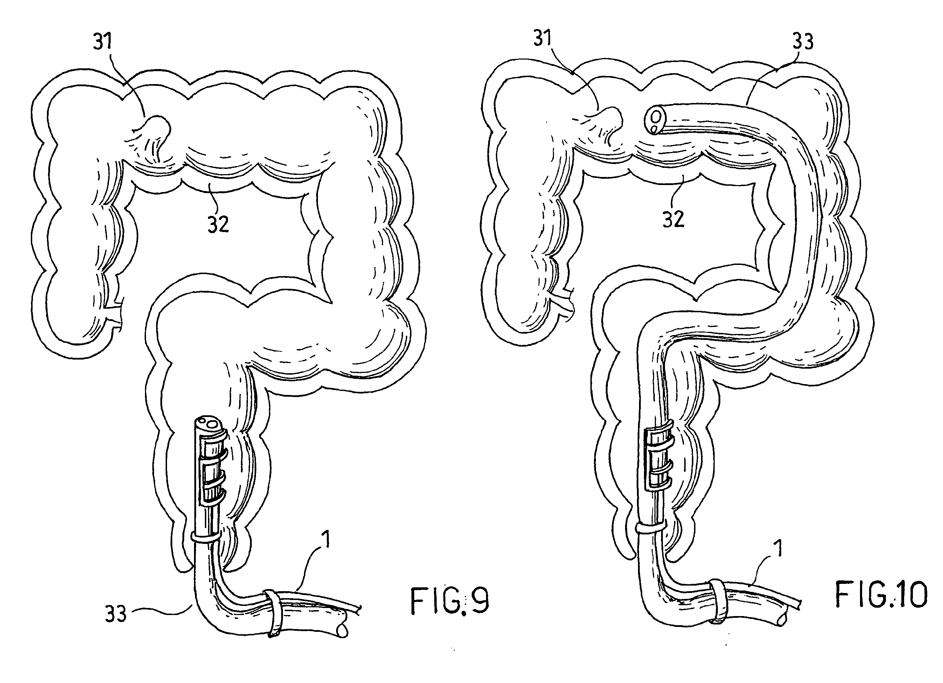

The step of accessing and clamping or invaginating the tissue comprises the substeps of:
- Inserting an endoscope 33, e.g. gastroscope or colonoscope, in order to identify the targeted tissue.
- Inserting the surgical tissue clamp 1 endolumenally up to the targeted tissue. Thanks to its curved clamping head 2, the surgical tissue clamp 1 can be inserted together with an endoscope 33 (Fig.9), slit along an endoscope (Fig. 10) or inserted alone through the natural duct or through a protective sheath previously placed in the natural duct.
- Inserting an endoscopic grasper 34 or forceps through a working channel 35 of the endoscope 33 up to the target site.
- Positioning the surgical tissue clamp 1 with open jaws 5, 6 over the lesion or polyp 31 to be excised, grasping the tissue with endoscopic forceps or grasper 34 and pulling it between the jaws of the clamping head 2, closing the jaws of the device by rotating the rotating knob 9 in order to hold the tissue in a clamped or invaginated configuration suitable for the following steps of tissue fixation and excision.

### Fixating and securing the tissue in the sense of a non-circumferential anastomosis

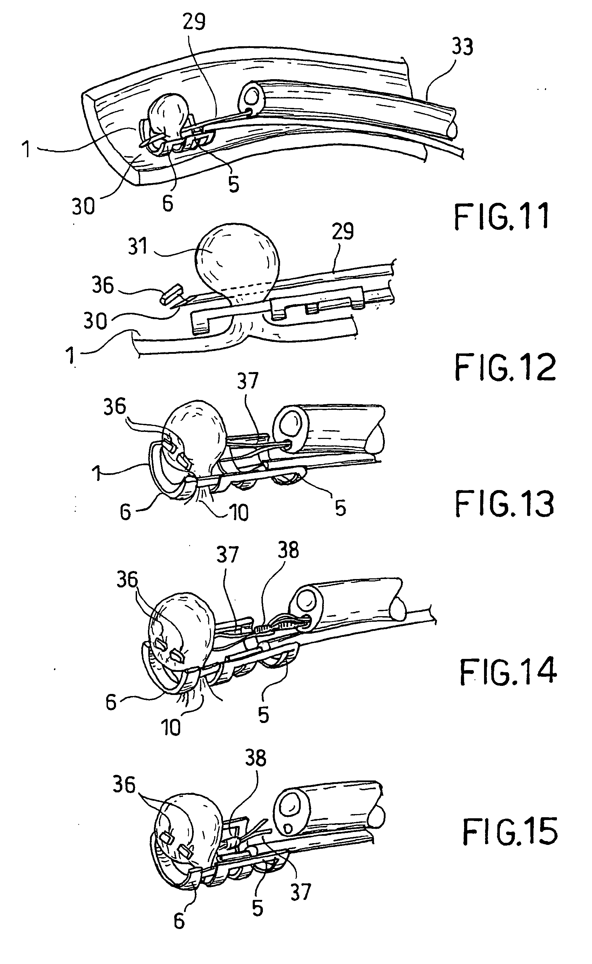

The step of fixating the tissue is performed after the tissue has been clamped and positioned by the tissue clamp 1 and comprises the substeps of:
- Inserting a tissue apposition device 29 through the working channel 35 of the endoscope 33 or through an additional accessory channel or along the outside of the endoscope directly through the natural duct or through a previously placed protective sheath up to the operational site.
- Piercing a needle end 30 of the tissue apposition device 29 distally through the full thickness of the invaginated polyp 31 and deploying a T-Tag fastener 36 with suture 37 through the needle end 30 so that the suture 37 protrudes proximally out of the invaginated tissue while the latter is held in position by the tissue clamp 1.
- Withdrawing the tissue apposition device 29 from the invaginated tissue 10, reloading the tissue apposition device 29 with a further T-Tag fastener 36 and applying the further T-Tag fastener 36 to the invaginated tissue as described above.
- With groups of at least two T-Tag fasteners 36 (Fig. 13) attached to the invaginated tissue 10, inserting a knotting device 38 to the operational site and knotting the sutures 37 of at least two T-Tag fasteners 36 of each group to another in order to tighten the T-Tag fasteners 36 and the suture 37 against the two apposed layers of tissue 10 to hold them compressed to one another. Also the knotting device 38 or knotting element, such as a suture clamp, is preferably introduced through a working channel 35 of the endoscope 33 or through an additional accessory channel or along the outside of the endoscope 33 directly through the natural duct or through a previously placed protective sheath.
- Repeating the application of T-Tag fasteners 36 and the knotting until a line of a non-circumferential anastomosis is provided with a sufficient tissue compression to promote healing.
- Removing the tissue apposition device 29 and the knotting device from the operational site (leaving the knots or knotting elements in place).

The tissue fixation is performed along the outside or inside of the clamping head 2. Figure 4 illustrates a tissue fixating step on the outside of the clamping head2, i.e. between the duct wall and the clamping head 2, while Figures 12 to 15 illustrate a tissue fixating step on the inside of the clamping head 2.

### Excision of the lesion or polyp

The step of excising the lesion or polyp 31 is performed after the tissue has been clamped and positioned by the tissue clamp 1 and fixated by instruments (tissue apposition device 29, T-Tag fasteners 36) different from the tissue clamp 1. The lesion or polyp 31 can now be excised by endolumenally inserting a cutting device, such as a radiofrequency snare or a needle knife 39, to the operational site. These cutting means are also preferably introduced through the working channel 35 of the endoscope 33 or through an additional accessory channel or along the outside of the endoscope directly through the natural duct or through a previously placed protective sheath.

The excision is preferably performed along the inside of the clamping head 2, leaving the fixating line with the fasteners 36 in place to keep the surgical site closed. After excision of the polyp 31 or lesion, the excised tissue sample and the surgical clamp 1 as well as the endoscope and the cutting device are removed from the operational site.

As will be immediately appreciated by those skilled in the art, the above described method and device provide a very versatile and cost-effective alternative to the all-in-one-device-approach of the prior art.

While the present invention has been illustrated by description of a preferred embodiment and while the illustrative embodiment has been described in considerable detail, it is not the intention to restrict or in any way limit the scope of the appended claims to such detail. Additional advantages and modifications may readily appear to those skilled in the art.

## Claims

1. A surgical tissue clamp (1), particularly for endolumenally clamping and invaginating tissue (10) during a polypectomy or excision of a lesion in general, comprising:
- a flexible elongate insertion shaft (4),
- a handle (3) arranged at a proximal end of the insertion shaft (4);
- a clamping head (2) arranged at a distal end of the insertion shaft (4) and including a proximal jaw (5) and a distal jaw (6) movable relative to the proximal jaw (5) and configured to cooperate with the proximal jaw (5) for clamping said tissue (10) in a substantially radial orientation with respect to a longitudinal axis (X) of the clamping head (2);
- an actuating device (8) operatively connected with said proximal and distal jaws (5, 6) and adapted to approximate said jaws and to hold said jaws in a tissue clamping configuration, whereby the proximal and distal jaws have a curved configuration
**characterized in that**
- the proximal jaw (5) comprises a curved proximal clamping segment (19) determining a tissue clamping line and a proximal spacing frame (20),
- the distal jaw (6) comprises a curved distal clamping segment (21) complementary to the proximal clamping segment (19) of the proximal jaw (5) and a distal spacing frame (22),
- said proximal spacing frame (20) and said proximal clamping segment (19) define a proximal access aperture (27) configured to access the space radially inside the jaws (5, 6) and the space radially outside the jaws (5, 6) directly across the clamping head (2),
- said distal spacing frame (22) is overlapping said proximal spacing frame (20) and defines a window opening (28) overlapping said proximal access aperture (27) in order not to obstruct the latter.

2. A surgical tissue clamp (1) according to claim 1, wherein:
- said curved proximal clamping segment (19) is connected to the insertion shaft (4) by said proximal spacing frame (20),
- said curved distal clamping segment (21)
is connected by means of said distal spacing frame (22) to a pulling rod (7) received by the insertion shaft (4) and operatively connected to the actuating device (8), wherein the curved proximal clamping segment (19) has a mean radial thickness of less than 1/8 of its bending radius, preferably less than 1/10 of its bending radius.

3. A surgical tissue clamp (1) according to claim 2, wherein the curved proximal clamping segment (19) has a longitudinal (proximal - distal) thickness of less than 1/2 of its bending radius, preferably less than 1/5 of its bending radius.

4. A surgical tissue clamp (1) according to claim 2 or 3, wherein the curved distal clamping segment (21) has a mean radial thickness of less than 1/8 of its bending radius, preferably less than 1/10 of its bending radius.

5. A surgical tissue clamp (1) according to any one of claims 2 to 4, wherein the curved distal clamping segment (21) has a longitudinal thickness of less than 1/2 of its bending radius, preferably less than 1/5 of its bending radius.

6. A surgical tissue clamp (1) according to any one of the preceding claims as dependent from claim 2, wherein the proximal spacing frame (20) comprises a substantially U-shaped frame having two longitudinal and approximately parallel opposite legs (23) distally connected to the proximal clamping segment (J9) and a curved proximal base spoke (24) connecting said proximal spacing frame (20) to the insertion shaft (4),
wherein the distal spacing frame (22) comprises a substantially U shaped or quadrangular frame having two longitudinal approximately parallel opposite legs (25) distally connected to the curved distal clamping segment (21) of the distal jaw (6) and a curved proximal base spoke (26) connecting said distal spacing frame (22) to the pulling rod,
wherein the longitudinal legs (25) of the distal spacing frame (22) are close to, preferably overlapping the longitudinal legs (23) of the proximal spacing frame (20).

7. A surgical tissue clamp (1) according to any of the preceding claims as dependent from claim 2, wherein the proximal and distal spacing frames (20, 22) are longitudinally straight and transversally curved forming a cylindrical halve shell frame or halfpipe frame defining internally the proximal access aperture (27).

8. A surgical tissue clamp (1) according to any one of the preceding claims,
wherein said actuating device (8) comprises:
- a manual actuating member (9);
- a moving mechanism (11, 12) operatively connected to the manual actuating member (9) and to the proximal and distal jaws (5, 6) and adapted to approximate and separate the jaws (5, 6) in response to an actuating movement of said manual actuating member (9),
- a locking mechanism (11, 12) operatively connected to the proximal and distal jaws (5, 6) and configured to lock the jaws (5, 6) in said tissue clamping configuration.

9. A surgical tissue clamp (1) according to the preceding claim, wherein said actuating device (8) comprises:
- a jackscrew (11) housed in a distal end portion of the insertion shaft (4) adjacent to the clamping head (2), said jackscrew (11) being rotatable but translationally locked with respect to the proximal jaw (5);
- a threaded connecting portion (12) of the pulling rod (7) of the distal jaw (6) meshing said jackscrew (11);
- a rotating knob (9) mounted on the handle (3) and torsionally coupled with the jackscrew (11) by a flexible but torsionally stiff rotary rod (13) received inside the insertion shaft (4) such that a rotation of the rotating knob (9) causes the jackscrew (11) to screw on the pulling rod (7) thereby approximating or separating the jaws (5, 6),
wherein the frictional resistance in said screw connection between the jackscrew (11) and the pulling rod (7) provides said jaw locking mechanism.

10. A surgical tissue clamp (1) according to any one of the preceding claims, wherein the clamping head (2) is detachably connected to the insertion shaft (4) enabling the single parts of the clamp (1) to be selectively disinfected, disposed and reused.

## Patentansprüche

1. Chirurgische Gewebeklemme (1), insbesondere zum endoluminalen Klemmen und Einstülpen von Gewebe (10) während einer Polypektomie oder allgemeinen Exzision einer Läsion, umfassend:
- einen flexiblen länglichen Einführungsschaft (4),
- einen an einem proximalen Ende des Einführungsschafts (4) angeordneten Griff (3);
- einen an einem distalen Ende des Einführungsschafts (4) angeordneten Klemmkopf (2), der eine proximale Klaue (5) und eine bezüglich der proximalen Klaue (5) bewegbare und zum Zusammenwirken mit der proximalen Klaue (5) zum Einklemmen des Gewebes (10) in einer im Wesentlichen radialen Ausrichtung bezüglich einer Längsachse (X) des Klemmkopfes (2) ausgebildete distale Klaue (6) umfasst;
- Stellvorrichtung (8), die betriebsmäßig mit der proximalen und der distalen Klaue (5, 6) verbunden und dazu ausgelegt ist, sich den Klauen zu nähern und die Klauen in einer Gewebeklemmkonfiguration zu halten, wodurch die proximale und die distale Klaue eine gekrümmte Konfiguration aufweisen, **dadurch gekennzeichnet, dass**
- die proximale Klaue (5) ein eine Gewebeklemmlinie bestimmendes gekrümmtes proximales Klemmsegment (19) und einen proximalen Abstandhalterahmen (20) umfasst,
- die distale Klaue (6) ein zu dem gekrümmten proximalen Klemmsegment (19) der proximalen Klaue (5) komplementäres gekrümmtes proximales Klemmsegment (21) und einen distalen Abstandhalterahmen (22) umfasst,
- der proximale Abstandhalterahmen (20) und das proximale Klemmsegment (19) eine proximale Zugangsöffnung (27) begrenzen, die für einen Zugang zu dem radial inneren Raum der Klauen (5, 6) und dem radial äußeren Raum der Klauen (5, 6) direkt über den Klemmkopf (2) ausgebildet ist,
- wobei der distale Abstandhalterahmen (22) den proximalen Abstandhalterahmen (20) überlappt und eine Fensteröffnung (28) begrenzt, die die proximale Zugangsöffnung (27) überlappt, um Letztere nicht zu blockieren.

2. Chirurgische Gewebeklemme (1) nach Anspruch 1, wobei:
- das gekrümmte proximale Klemmsegment (19) mit dem Einführungsschaft (4) durch den proximalen Abstandhalterahmen (20) verbunden ist,
- das gekrümmte proximale Klemmsegment (21) durch den distalen Abstandhalterahmen (22) mit einer in dem Einführungsschaft (4) aufgenommenen Zugstange (7) verbunden ist und mit der Stellvorrichtung (8) betriebsmäßig verbunden ist,
wobei das gekrümmte proximale Klemmsegment (19) eine mittlere radiale Dicke von weniger als 1/8 seines Biegeradius, vorzugsweise weniger als 1/10 seines Biegeradius aufweist.

3. Chirurgische Gewebeklemme (1) nach Anspruch 2, wobei das gekrümmte proximale Klemmsegment (19) eine Dicke in Längsrichtung (proximal - distal) von weniger als 1/2 seines Biegeradius, vorzugsweise weniger als 1/5 seines Biegeradius aufweist.

4. Chirurgische Gewebeklemme (1) nach Anspruch 2 oder 3, wobei das gekrümmte distale Klemmsegment (21) eine mittlere radiale Dicke von weniger als 1/8 seines Biegeradius, vorzugsweise 1/10 seines Biegeradius aufweist.

5. Chirurgische Gewebeklemme (1) nach einem der Ansprüche 2 bis 4, wobei das gekrümmte distale Klemmsegment (21) eine Dicke in Längsrichtung von weniger als 1/2 seines Biegeradius, vorzugsweise weniger als 1/5 seines Biegeradius aufweist.

6. Chirurgische Gewebeklemme (1) nach einem der vorangehenden Ansprüche, sofern abhängig von Anspruch 2, wobei der proximale Abstandhalterahmen (20) einen im Wesentlichen U-förmigen Rahmen umfasst, der zwei in Längsrichtung verlaufende und ungefähr parallel, gegenüberliegende Schenkel (23) umfasst, die distal mit dem proximalen Klemmsegment (19) verbunden sind, und eine gekrümmte proximale Grundspeiche (24) umfasst, die den proximalen Abstandhalterahmen (20) mit dem Einführungsschaft (4) verbindet,
wobei der distale Abstandhalterahmen (22) einen im Wesentlichen U-förmigen oder rechteckigen Rahmen mit zwei in Längsrichtung verlaufenden und ungefähr parallelen, gegenüberliegenden Schenkeln (25) umfasst, die distal mit dem gekrümmten distalen Klemmelement (21) der distalen Klaue (6) verbunden sind, und eine gekrümmte proximale Grundspeiche (26) umfasst, die den distalen Abstandhalterahmen (22) mit der Zugstange verbindet, wobei sich die in Längsrichtung verlaufenden Schenkel (25) des distalen Abstandhalterahmens (22) in der Nähe der in Längsrichtung verlaufenden Schenkel (23) des proximalen Abstandhalterahmens (20) befinden oder ihn überlagern.

7. Chirurgische Gewebeklemme (1) nach einem der vorangehenden Ansprüche sofern abhängig von Anspruch 2, wobei der proximale und der distale Abstandhalterahmen (20, 22) in Längsrichtung gerade und in Querrichtung gekrümmt sind und einen zylindrischen Halbschalenrahmen oder einen Halfpipe-Rahmen bilden, der im Inneren die proximale Zugangsöffnung (27) begrenzt.

8. Chirurgische Gewebeklemme (1) nach einem der vorangehenden Ansprüche, wobei die Stellvorrichtung (8) umfasst:
- ein manuelles Stellelement (9);
- einen Bewegungsmechanismus (11, 12), der betriebsmäßig mit dem manuellen Stellelement (9) und der proximalen und der distalen Klaue (5, 6) verbunden ist und dazu ausgelegt ist, die Klauen (5, 6) in Reaktion auf eine Stellbewegung des manuellen Stellelements (9) anzunähern und zu trennen,
- einen Arretierungsmechanismus (11, 12), der betriebsmäßig mit der proximalen und der distalen Klaue (5, 6) verbunden ist und dazu ausgelegt ist, die Klauen (5, 6) in der Gewebeklemmkonfiguration zu arretieren.

9. Chirurgische Gewebeklemme (1) nach dem vorangehenden Anspruch, wobei die Stellvorrichtung (8) umfasst:
- eine in einem distalen Endabschnitt des Einführungsschafts (4) benachbart zu dem Klemmkopf (2) aufgenommene Spindel (11), wobei die Spindel (11) drehbar, aber verschiebemäßig bezüglich der proximalen Klaue (5) arretiert ist;
- einen mit Gewinde versehenden Verbindungsabschnitt (12) der Zugstange (7) der distalen Klaue (6), der mit der Spindel (11) eingreift;
- einen an dem Griff (3) angebrachten Drehknopf (9), der mit der Spindel (11) durch eine flexible, aber torsionssteife Drehstange (13) drehgekoppelt ist, welche in dem Einführungsschaft (4) aufgenommen ist, so dass eine Drehung des Drehknopfes (9) bewirkt, dass sich die Spindel (11) auf die Zugstange (7) schraubt, wodurch die Klauen (5, 6) aneinander angenähert oder voneinander getrennt werden,
wobei der Reibungswiderstand in der Schraubverbindung zwischen der Spindel (11) und der Zugstange (7) den Klauenarretierungsmechanismus vorsieht.

10. Chirurgische Gewebeklemme (1) nach einem der vorangehenden Ansprüche, wobei der Klemmkopf (2) lösbar mit dem Einführungsschaft (4) verbunden ist und ermöglicht, dass die Einzelteile der Klemme (1) selektiv desinfiziert, entsorgt und wiederverwendet werden können.

## Revendications

1. Pince chirurgicale (1) pour tissus, particulièrement pour pincer des tissus par voie endoluminale et les retrousser pendant une polypectomie ou une excision d'une lésion en général, comprenant :
- une tige d'insertion allongée flexible (4),
- une manette (3) agencée à une extrémité proximale de la tige d'insertion (4) ;
- une tête de pince (2) agencée à une extrémité distale de la tige d'insertion (4) et incluant une mâchoire proximale (5) et une mâchoire distale (6) mobile par rapport à la mâchoire proximale (5) et configurée pour coopérer avec la mâchoire proximale (5) pour pincer lesdits tissus (10) dans une orientation sensiblement radiale par rapport à un axe longitudinal (X) de la tête de pince (2) ;
- un dispositif d'actionnement (8) fonctionnellement connecté à ladite mâchoire proximale et ladite mâchoire distale (5, 6) et adapté à rapprocher lesdites mâchoires et à tenir lesdites mâchoires dans une configuration ou elles pincent des tissus, dans laquelle la mâchoire proximale et la mâchoire distale ont une configuration incurvée, **caractérisée en ce que**
- la mâchoire proximale (5) comprend un segment de pince proximal incurvé (19) déterminant une ligne de pincement des tissus, et un cadre d'écartement proximal (20),
- la mâchoire distale (6) comprend un segment de pince distal incurvé (21) complémentaire du segment de pince proximal (19) de la mâchoire proximale (5) et un cadre d'écartement distal (22),
- ledit cadre d'écartement proximal (20) et ledit segment de pince proximal (19) définissent une ouverture d'accès proximale (27) configurée pour l'accès à l'espace radialement à l'intérieur des mâchoires (5, 6) et à l'espace radialement à l'extérieur des mâchoires (5, 6) directement à travers la tête de pince (2),
- ledit cadre d'écartement distal (22) chevauche ledit cadre d'écartement proximal (20) et définit une ouverture de fenêtre (28) qui chevauche ladite ouverture d'accès proximale (27) afin de ne pas obstruer cette dernière.

2. Pince chirurgicale (1) pour tissus selon la revendication 1, dans laquelle :
- ledit segment de pince proximal incurvé (19) est connecté à la tige d'insertion (4) par ledit cadre d'écartement proximal (20),
- ledit segment de pince distal incurvé (21) est connecté au moyen dudit cadre d'écartement distal (22) à une barre de traction (7) reçue par la tige d'insertion (4) et fonctionnellement connectée au dispositif d'actionnement (8),
dans laquelle le segment de pince proximal incurvé (19) présente une épaisseur radiale moyenne inférieure à 1/8 de son rayon de courbure, de préférence inférieure à 1/10 de son rayon de courbure.

3. Pince chirurgicale (1) pour tissus selon la revendication 2,
dans laquelle le segment de pince proximal incurvé (19) présente une épaisseur longitudinale (proximale-distale) inférieure à 1/2 de son rayon de courbure, de préférence inférieure à 1/5 de son rayon de courbure.

4. Pince chirurgicale (1) pour tissus selon la revendication 2 ou 3, dans laquelle le segment de pince distal incurvé (21) présente une épaisseur radiale moyenne inférieure à 1/8 de son rayon de courbure, de préférence inférieure à 1/10 de son rayon de courbure.

5. Pince chirurgicale (1) pour tissu selon l'une quelconque des revendications 2 à 4, dans laquelle le segment de pince distal incurvé (21) présente une épaisseur longitudinale inférieure à 1/2 de son rayon de courbure, de préférence inférieure à 1/5 de son rayon de courbure.

6. Pince chirurgicale (1) pour tissus selon l'une quelconque des revendications précédentes, prise en dépendance de la revendication 2, dans laquelle le cadre d'écartement proximal (20) comprend un cadre sensiblement en forme de U ayant deux branches longitudinales opposées (23) approximativement parallèles, connectées en situation distale au segment de pince proximal (19), et un bras de base proximal incurvé (24) qui connecte ledit cadre d'écartement proximal (20) à la tige d'insertion (4),
dans laquelle le cadre d'écartement distal (22) comprend un cadre sensiblement en forme de U ou un cadre rectangulaire ayant deux branches opposées longitudinales (25) approximativement parallèles connectées en situation distale au segment de pince distal incurvé (21) de la mâchoire distale (6) et un bras de base proximal incurvé (26) qui connecte ledit cadre d'écartement distal (22) à la barre de traction,
dans laquelle les branches longitudinales (25) du cadre d'écartement distal (22) sont proches des branches longitudinales (23), de préférence en chevauchement de celles-ci, du cadre d'écartement proximal (20).

7. Pince chirurgicale (1) pour tissus selon l'une quelconque des revendications précédentes prise en dépendance de la revendication 2, dans laquelle le cadre d'écartement proximal et le cadre d'écartement distal (20, 22) sont longitudinalement rectilignes et incurvés transversalement en formant un cadre en forme de demi-coque ou de demi-tube cylindrique qui définit à l'intérieur l'ouverture d'accès proximale (27).

8. Pince chirurgicale (1) pour tissus selon l'une quelconque des revendications précédentes,
dans laquelle ledit dispositif d'actionnement (8) comprend :
- un élément d'actionnement manuel (9) ;
- un mécanisme de déplacement (11, 12) fonctionnellement connecté à l'élément d'actionnement manuel (9) et à la mâchoire proximale et la mâchoire distale (5, 6) et adapté à rapprocher et à séparer les mâchoires (5, 6) en réponse à un mouvement d'actionnement dudit élément d'actionnement manuel (9),
- un mécanisme de blocage (11, 12) fonctionnellement connecté à la mâchoire proximale et à la mâchoire distale (5, 6) et configuré pour bloquer les mâchoires (5, 6) dans ladite configuration de pincement de tissus.

9. Pince chirurgicale (1) pour tissus selon la revendication précédente, dans laquelle ledit dispositif d'actionnement (8) comprend :
- une vis-vérin (11) logée dans une portion d'extrémité distale de la tige d'insertion (4) adjacente à la tête de pince (2), ladite vis-vérin (11) étant capable de rotation mais bloquée en translation par rapport à la mâchoire proximale (5) ;
une portion de connexion vissée (12) de la barre de traction (7) de la mâchoire distale (6) en engagement avec ladite vis-vérin (11) ;
- un bouton rotatif (9) monté sur la manette (3) et couplé en torsion avec la vis-vérin (11) par une barre rotative flexible mais rigide en torsion (13) reçue à l'intérieur de la tige d'insertion (4) de telle façon qu'une rotation du bouton rotatif (9) amène la vis-vérin (11) à se visser sur la barre de traction (7) en rapprochant ainsi ou en séparant les mâchoires (5, 6),
dans laquelle la résistance à la friction dans ladite connexion vissée entre ladite vis-vérin (11) et la barre de traction (7) constitue ledit mécanisme de blocage de mâchoire.

10. Pince chirurgicale (1) pour tissus selon l'une quelconque des revendications précédentes, dans laquelle la tête de pince (2) est connectée de manière détachable à la tige d'insertion (4) permettant de désinfecter, de jeter et de réutiliser de manière sélective les parties individuelles de la pince (1).
